# EUROPEAN PATENT SPECIFICATION

(11) **EP 1 381 338 B1**
(45) Date of publication and mention of the grant of the patent: **23.08.2006**
(21) Application number: 01273959.5
(22) Date of filing: 09.11.2001
(51) Int. Cl.: A61F 9/007, A61F 9/011

(54) **LACRIMAL DUCT ENDOSCOPY UNIT**
GERÄT FÜR DIE ENDOSKOPIE DES TRÄNENKANALS
UNITE D'ENDOSCOPIE PAR VOIE LACRYMALE

(30) Priority: 13.03.2001 IT MC20010027
(43) Date of publication of application: 21.01.2004
(73) Proprietor: OPTOSGROUP S.p.A., 00195 Roma (IT)
(72) Inventor: VALAZZI, Carlo, Maria, I-61100 Pesaro (IT)
(74) Representative: Jorio, Paolo
(86) International application number: PCT/EP2001/013004
(87) International publication number: WO 2002/071991

(56) References cited:
- DE-A- 19 542 955
- US-A- 3 809 093
- US-A- 3 945 375
- US-A- 4 607 622

## Description

The present invention relates to a handset for lacrimal duct endoscopy unit.

In other words, the present invention relates to a handset for endoscopic inspection and surgical operation of lacrimal ducts.

In US 4 607 622, for instance, it is described an ocular endoscope having a minimum cross-section and designed primarily for use in treating, diagnosing and investigating problems associated with the eye. The probe associated with the endoscope comprises a first bundle of fiber optics carrying light for illumination, a second coaxial bundle of fiber optics terminating in a lens and adapted to view areas being illuminated also included within this known endoscope is a conduit located coaxial with the optical fibers bundle and filled with fiber optics that allow laser surgery. The conduit may be used for accepting cutting devices.

The innovative handset described allows a specialist operator to inspect and, if necessary, perform surgical operations along the lacrimal duct, with the operating point kept under continuous observation.

The handset according to the present invention provides for visually detecting and surgically operating any pre-sac stenosis, while at the same time guiding the reopening of post-sac occlusions.

It is object of the present invention therefore to provide a handset as claimed in Claim 1.

A non-limiting embodiment of the present invention will be described by way of example with reference to the accompanying drawings, in which:
Figure 1 shows, as a whole, a unit designed to implement a corresponding method of inspecting a lacrimal duct and, if necessary, surgically operating any occlusions therein;
Figure 2 shows an embodiment of a handset, object of the present invention, which is a device employed in conjunction with the Figure 1 unit;
Figure 3 shows a configuration of a handset, also employed in conjunction with the Figure 1 unit;
Figure 4 shows an exploded view of the Figure 3 handset.

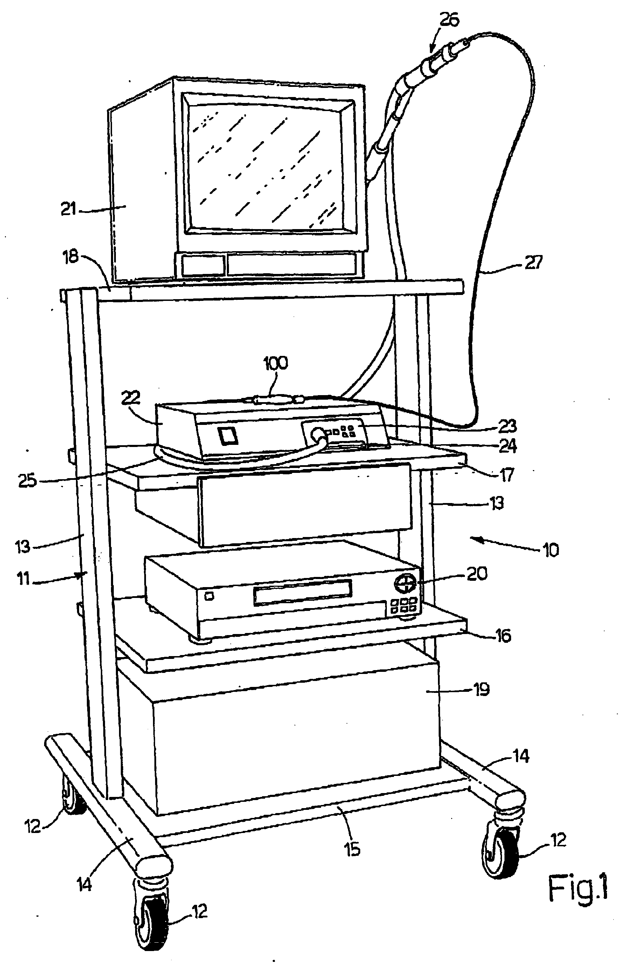

Number 10 in Figure 1 indicates as a whole a lacrimal duct endoscopy unit.

The unit 10 comprises a trolley 11 mounted on four castors 12 (only three shown in Figure 1) and in turn comprising two uprights 13, and two cross members 14 fitted with castors 12.

Trolley 11 also comprises four shelves 15, 16, 17, 18 supported by the structure defined by uprights 13 and cross members 14.

Shelf 15 supports a mains isolator 19 extraneous to the present invention and therefore not described in detail.

Shelf 16 supports a conventional video recorder 20 connected electrically (the electric connections not shown) to a known monitor 21 on the top shelf 18 of trolley 11.

Shelf 17 supports a conventional lighting device 22 for supplying a polarized-light beam along an optical fiber (not shown in Figure 1).

Lighting device 22 comprises a pull-out video camera 23 with a handle 24 by which to extract it from a seat (not shown) formed in the body of device 22.

From video camera 23 extends a cable 25 of the type housing both an optical fiber (not shown) and a supply cable (not shown) for powering video camera 23. One end of cable 25 is fitted with a connecting device 26 for connection to a sheathed optical fiber 27 for illuminating a handset 100 of the type described in detail later on.

As described in detail later on, one portion of handset 100 is inserted endoscopically inside a lacrimal duct to optically inspect the duct and, if necessary, surgically operate any occlusions in the duct.

Operation of unit 10 is self-explanatory:
- after turning on unit 10, a specialist operator simply inserts the appropriate portion of handset 100 (see below) into the lacrimal duct;
- monitor 21 immediately displays enlarged images of the portion of the lacrimal duct housing the end portion of optical fiber 27 extending through handset 100 (see below);
- the operator assesses the gravity of any occlusions detected, and immediately decides whether or not to operate surgically on the occlusion;
- if so, the operator operates surgically on the occlusion using handset 100 itself; and
- the operator examines the outcome of the operation, again using handset 100.

Examination and surgery may, obviously, be performed synchronously.

If necessary, the film shot by video camera 23 may be recorded on video recorder 20.

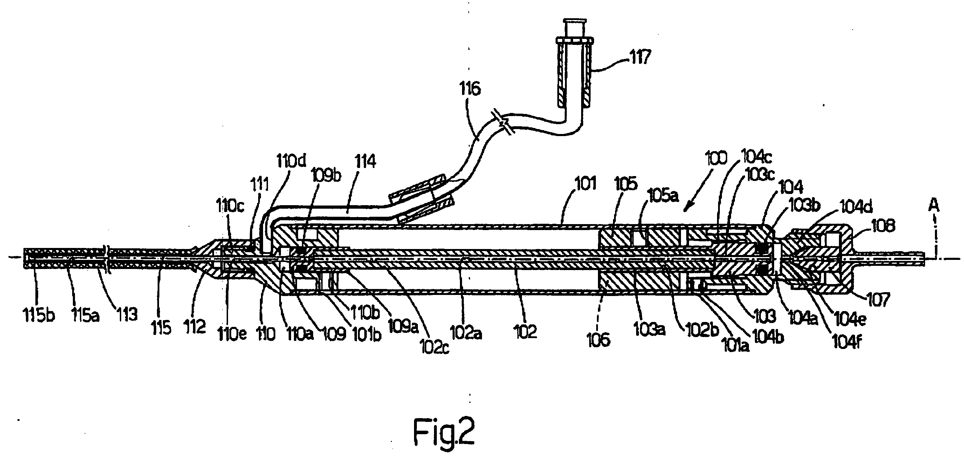

Figure 2 shows an embodiment of handset 100, which is an object of the present invention.

Handset 100 comprises a tubular body 101 housing an axially-sliding spindle 102, through which extends longitudinally an axial conduit 102a housing optical fiber 27 (Figure 1).

Handset 100 is substantially symmetrical with respect to an axis A.

A rear portion 102b of spindle 102 is fitted inside a nozzle 103a forming an integral part of a rear support 103 of spindle 102. Rear support 103 is substantially piston-shaped, is fitted with a seal 103b, and slides axially, in the direction defined by axis A, inside a cylindrical chamber 104a formed in an endpiece 104 closing the rear of body 101.

A hole 101a is provided close to the rear end of body 101, and corresponds with a threaded hole 104b formed in the wall of endpiece 104 and in which a radial pin (not shown) is screwed to lock endpiece 104 axially. When screwed right down, the tip of the radial pin interferes with nozzle 103a to limit the travel of spindle 102.

Rear support 103 and endpiece 104 are connected by means of a screw coupling 104c.

Spindle 102 can be slid forwards or backwards manually by the operator gripping, between two fingers, a collar 105 connected integrally to spindle 102 by a radial fastening pin (not shown) screwed inside a threaded hole 105a formed in collar 105. To enable the operator's fingers to act on collar 105, and hence on spindle 102, body 101 is provided with two opposite windows 106.

Endpiece 104 terminates at the rear with an externally threaded appendix 104d having a conical seat 104e housing a cone 107 for fastening optical fiber 27 (Figure 1); and cone 107 is locked inside conical seat 104e by a ring nut 108 screwed on to the outside of appendix 104d.

Endpiece 104 obviously comprises an axial through conduit 104f for the passage of optical fiber 27, and which is obviously aligned perfectly with a conduit 103c formed in support 103 and interfaced directly with axial conduit 102a in spindle 102.

A front portion 102c of spindle 102 is fitted inside a nozzle 109a forming an integral part of a front support 109 of spindle 102. Front support 109 is also substantially piston-shaped, is fitted with a seal 109b, and slides, in the direction defined by axis A, inside a cylindrical chamber 110a formed in an endpiece 110 closing the front of body 101.

In other words, endpieces 104 and 110 close the rear end and front end of body 101 respectively.

A hole 101b is provided close to the front end of body 101, and corresponds with a threaded hole 110b formed in the wall of endpiece 110, which, as stated, is inserted inside the open front end of body 101; and a second threaded pin (not shown) is screwed inside hole 110b to prevent any axial movement of endpiece 110.

At the front, endpiece 110 comprises an externally threaded, substantially cylindrical appendix 110c.

With the interposition of a seal 111, a special ring nut 112 is screwed on to appendix 110c and supports a cannula 113 for insertion inside a lacrimal duct (not shown).

Endpiece 110 also has a lateral hole 110d, in which is fitted a rigid tube 114 for feeding irrigation fluid into a through conduit 110e extending axially through endpiece 110. The irrigation fluid flows into cannula 113, which houses a drilling tool 115 rotated and fed axially forward by the rotation and axial feed of spindle 102, with which drilling tool 115 is integral.

A through conduit 115a extends longitudinally through drilling tool 115, and houses optical fiber 27 (not shown in Figure 2), which, to illuminate and pick up images of the lacrimal duct, must extend up to the end 115b of drilling tool 115.

Rigid tube 114 is connected to a flexible tube 116 fitted on the end with a bracket 117 for fast fit and removal of a syringe (not shown) containing the lacrimal duct irrigation fluid.

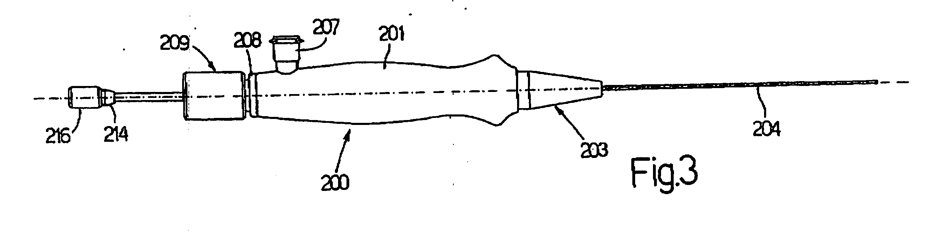

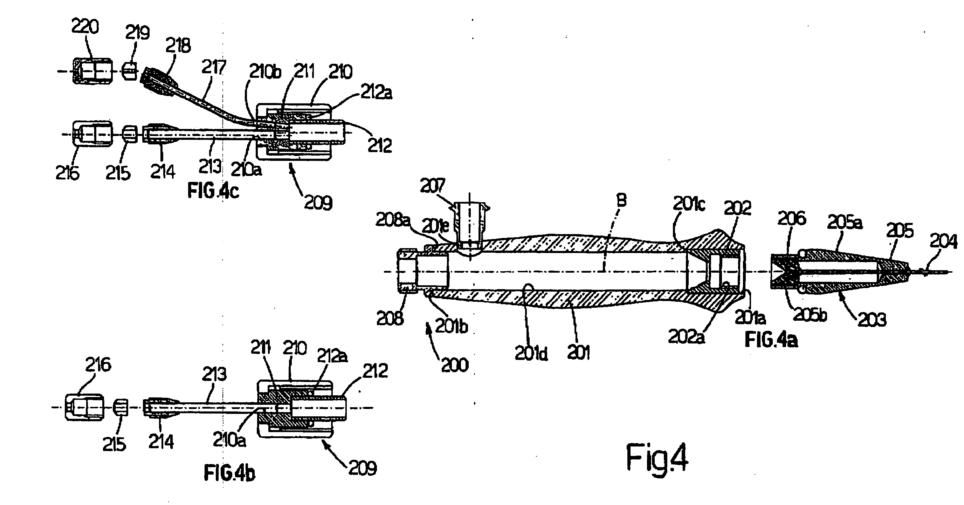

Figures 3 and 4 show a configuration of a handset 200 which does not fall within the scope of the appended claims.

Handset 200 comprises a substantially tubular body 201 (Figure 4a) symmetrical with respect to a longitudinal axis B, having two opposite openings 201a, 201b, and shaped for improved grip of handset 200 by the operator.

An endpiece 202 is inserted inside opening 201a up to a shoulder 201c formed on the wall of a through conduit 201d extending longitudinally through body 201, from opening 201a to opening 201b.

Endpiece 202 is fixed to body 201 in the same way as in the first embodiment shown in Figure 2, i.e. by means of a fastening pin (not shown), which fits through a hole (not shown) in body 201, and screws into a threaded hole (not shown) in endpiece 202.

Inside, endpiece 202 also has a longitudinal conduit 202a, which is also coaxial with axis B, and at least one portion of which is threaded to screw on a supporting member 203 for a bored tool 204. More specifically, supporting member 203 comprises an outer shell 205 in which is inserted a bored bushing 206.

As shown in detail in Figure 4a, bored tool 204 is therefore supported at one end by outer shell 205, and at the other end by bored bushing 206. Outer shell 205 can be divided theoretically into a substantially truncated-cone-shaped first portion 205a, and a cylindrical second portion 205b having an external thread by which, as stated, to screw supporting member 203 to endpiece 202.

Body 201 also has a lateral opening 201e through which to supply irrigation fluid in the same way as in Figure 2.

A further endpiece 208 is inserted inside opening 201b, and has a shoulder 208a which mates with the edge of opening 201b.

At least one portion of endpiece 208 projecting from body 201 has a thread for the reasons explained in detail later on.

Figures 4b and 4c show two further alternative configurations of handset 200.

The first configuration in Figure 4b employs only one optical fiber (not shown) for simply detecting optically the condition of the lacrimal duct, while the second embodiment in Figure 4c employs a second optical fiber (not shown) for conveying a laser beam produced by a generator (not shown).

More specifically, and firstly with reference to both Figures 4a and 4b, body 201 is closed at opening 201b by a closing device 209.

Closing device 209 comprises a bored, internally threaded ring nut 210, a bored plug 211 inserted inside ring nut 210, and a tube 212 surrounded by a sealing ring 212a, and, by means of the internal thread of ring nut 210, screws on to the external thread of endpiece 208.

Ring nut 210 has a hole 210a through which is inserted a tube 213 for insertion of the optical fiber (not shown), which, in this case, as stated, merely serves to determine the condition of the lacrimal duct optically.

The optical fiber is fixed firmly using a bored cone 214 partly threaded on the outside and which fits on to tube 213; a spacer member 215; and an internally threaded ring nut 216 for receiving spacer member 215 and which screws on to the threaded portion of cone 214.

As stated, in addition to the devices housing and supporting a first image-detecting optical fiber, the Figure 4c configuration is also designed to insert inside handset 200 a second optical fiber (not shown) for conveying a laser beam by which to remove any occlusions detected in the lacrimal duct.

Consequently, in addition to hole 210a, plug 211 also has a further hole 210b in which is inserted a respective tube 217.

As before, tube 217 is provided with a cone 218, a spacer member 219, and an internally threaded ring nut 220 for receiving spacer member 219 and which screws on to the threaded portion of cone 218.

By means of the second solution shown in Figures 4a and 4c, the operator, on detecting an occlusion, removes it using a laser beam conveyed by the second optical fiber (not shown) through tube 217.

In a further configuration (not shown) a handset comprises trigger means for shooting forward a punching tool to remove particularly tough occlusions.

## Claims

1. A handset (100) for lacrimal duct endoscopy, the handset (100) comprising means (27) for optically detecting occlusions in the duct, said means for optically detecting occlusions comprising optical fiber (27), and means (115) for surgically removing the occlusions , said means for surgically removing the occlusions comprising a mechanical drilling tool (115) and a feed device (102) to feed, during use, the mechanical drilling tool forward axially, said mechanical drilling tool (115) being provided with a through conduit (115a) extending longitudinally through said drilling tool (115) and housing said optical fiber (27).

2. A handset (100) as claimed in Claim 1, further comprising a punching tool adapted to shoot axially forward by trigger means to remove the occlusion.

3. A handset (100) as claimed in Claim 1, further comprising a cannula (113) housing said drilling tool (115) for insertion inside a lacrimal duct.

4. A handset (100)as claimed in Claim 3, wherein an endpiece (110) of the handset (100) has a lateral hole (110d), in which is fitted a rigid tube (114) for feeding irrigation fluid into a through conduit (110e) extending axially through the endpiece (110), the irrigation fluid flowing into the cannula (113).

## Patentansprüche

1. Handgerät (100) für die Endoskopie des Tränenkanals mit Mitteln (27) zur optischen Feststellung von Verstopfungen in diesem Kanal, wobei die genannten Mittel zur optischen Feststellung von Verstopfungen einen Lichtleiter (27) umfassen sowie Mittel (115) zur chirurgischen Entfernung der Verstopfungen, wobei die genannten Mittel zur chirurgischen Entfernung der Verstopfungen ein mechanisches Bohrwerkzeug (115) und eine Zuführeinrichtung (102) umfassen, um während des Gebrauchs das mechanische Bohrwerkzeug axial in einer Vorwärtsrichtung zuzustellen, wobei das genannte mechanische Bohrwerkzeug (115) mit einer Durchgangsleitung (115a) versehen ist, die sich in Längsrichtung durch das genannte Bohrwerkzeug (115) hindurcherstreckt und den genannten Lichtleiter (27) aufnimmt.

2. Handgerät (100) nach Anspruch 1, welches ferner ein Stanzwerkzeug umfasst, welches dazu bestimmt ist, mittels Steuermitteln in Vorwärtsrichtung gestoßen zu werden, um die Verstopfungen zu entfernen.

3. Handgerät (100) nach Anspruch 1, welches ferner eine Kanüle (113) umfasst, in der das genannte Bohrwerkzeug (115) aufgenommen ist und die dazu bestimmt ist, in einen Tränenkanal eingesetzt zu werden.

4. Handgerät (100) nach Anspruch 3, wobei ein Endstück (110) des Handgerätes (100) eine seitliche Öffnung (110d) aufweist, in welche ein starres Rohr (114) eingesetzt ist, welches dazu bestimmt ist, eine Spülflüssigkeit in eine Durchgangsleitung (110e) einzuführen, welche sich axial durch das Endstück (110) hindurch erstreckt, wobei die Spülflüssigkeit in die Kanüle (113) fließt.

## Revendications

1. Unité d'endoscopie (100) pour l'endoscopie du canal lacrymal, l'unité d'endoscopie (100) comprenant un moyen (27) pour détecter optiquement les occlusions dans le canal, ledit moyen pour détecter optiquement les occlusions comprenant des fibres optiques (27), pour éliminer chirurgicalement les occlusions, ledit moyen pour éliminer chirurgicalement les occlusions comprenant un foret mécanique (115) et un dispositif d'alimentation (102) pour alimenter pendant l'utilisation, le foret mécanique avancé axialement, ledit foret mécanique (115) étant doté un conduit traversant (115a) s'étendant longitudinalement au travers dudit foret (115) et un logement pour lesdites fibres optiques (27).

2. Unité d'endoscopie (100) selon la revendication 1, comprenant en outre un outil de découpage adapté pour être propulsé axialement par un déclencheur pour éliminer l'occlusion.

3. Unité d'endoscopie (100) selon la revendication 1, comprenant en outre une canule (113) abritant ledit foret (115) pour insertion dans le canal lacrymal.

4. Unité d'endoscopie (100) selon la revendication 3, dans lequel une pièce terminale (110) de l'unité d'endoscopie (100) présente un alésage latéral (110d) dans lequel est fixé un tube rigide (114) pour apporter un fluide d'irrigation dans un conduit traversant (110e) s'étendant axialement au travers de la pièce terminale (110), le fluide d'irrigation s'écoulant dans la canule (113).
